# EUROPEAN PATENT APPLICATION

(11) **EP 4 108 244 A1**
(43) Date of publication of application: **28.12.2022**
(21) Application number: 21181839.8
(22) Date of filing: 25.06.2021
(51) Int. Cl.: A61K 31/513, A61K 31/546, A61P 35/00, A61K 45/06

(54) **SS-LACTAM ANTIBIOTIC WITH SIGNIFICANT ACTIVITY AGAINST CANCER E.G. COLON MALIGNANCIES**

(71) Applicant: Universität Regensburg, 93053 Regensburg (DE)
(72) Inventor: EL-NAJJAR, Nahed., 93049 Regensburg (DE); GESSNER, André., 93053 Regensburg (DE)
(74) Representative: Engelhard, Markus

(57) **Abstract**

The present invention relates to a cephalosporin for use in a method of preventing and/or treating colon cancer, wherein the cephalosporin comprises a nitrogen, preferably positively charged nitrogen, and a first carboxyl group, preferably a negatively charged carboxyl group; wherein the nitrogen and the carboxyl group of said cephalosporin are spaced apart by three carbon atoms. Furthermore, the present invention relates to a pharmaceutical composition for use in a method of preventing and/or treating colon cancer, wherein said composition comprises a cephalosporin and a pharmaceutically acceptable excipient.

## Description

### FIELD OF THE INVENTION

The present invention relates to a cephalosporin for use in a method of preventing and/or treating colon cancer, wherein the cephalosporin comprises a nitrogen, preferably positively charged nitrogen, and a first carboxyl group, preferably a negatively charged carboxyl group; wherein the nitrogen and the carboxyl group of said cephalosporin are spaced apart by three carbon atoms. Furthermore, the present invention relates to a pharmaceutical composition for use in a method of preventing and/or treating colon cancer, wherein said composition comprises a cephalosporin and a pharmaceutically acceptable excipient.

### BACKGROUND OF THE INVENTION

According to the world health organization, cancer-remains a leading cause of death worldwide, accounting for 9.6 million deaths in 2018. Colon cancer is the most common gastrointestinal cancer and the second leading cause of cancer deaths in the world. The inactivation of different types of regulatory genes including tumor suppressor genes, promoncogenes and DNA mismatch repair genes are the basis of colon cancer initiation and progression. Maintaining the balance between cell proliferation and apoptosis in colonic epithelial cells is essential for the preservation of the constant crypt proliferation, differentiation and cell death. Consequently, alteration of different players preserving this balance is one of the earliest events in colorectal carcinogenesis.

In early stages of colon cancer, the disease is often treated by surgery. In later stages, typically cytostatic agents or other tumor growth inhibiting compounds are administered. In later stages, in addition to chemotherapy and radiotherapy, targeted therapeutics such as bevacizumab, cetuximab, panitumumab and regorafenib are commonly prescribed; however, the latter agents are mainly used in developed countries due to high costs. Additional treatment approaches are needed. Particularly, for emerging market countries and developing countries, inexpensive active agents are needed that are effective in treating colon cancer.

Most of the current treatments of early-stage cancer rely on suppressing cell proliferation. The current treatments typically do not differentiate between normal and cancer cells, hence, such treatments have many side effects. For example, 5-fluorouracil is a standard therapy for colon cancer and typically evokes side effects. Thus, there is a need for drugs devoid of side effects or with limited side effects. Furthermore, there is a need to identify compounds having a therapeutic effect against colon cancer. There is also a need for effective treatments against colon cancers characterized by 5-fluorouracil resistance, e.g. cancers comprising 5-fluorouracil resistant cancer cells.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a cephalosporin for use in a method of preventing and/or treating colon cancer, wherein the cephalosporin comprises a nitrogen, preferably positively charged nitrogen, and a first carboxyl group, preferably a negatively charged carboxyl group; wherein the nitrogen and the carboxyl group of said cephalosporin are spaced apart by three carbon atoms;
wherein, optionally, said cephalosporin further comprises a second carboxyl group;
wherein, preferably, the cephalosporin is a third-generation cephalosporin or fourth-generation cephalosporin.

In one embodiment, the cephalosporin is selected from ceftazidime, cefpimizole, cefiderocol, ceftolozane, and derivatives thereof, in particular pharmaceutically acceptable salts, solvates, and hydrates thereof.

In one embodiment, the cephalosporin is selected from 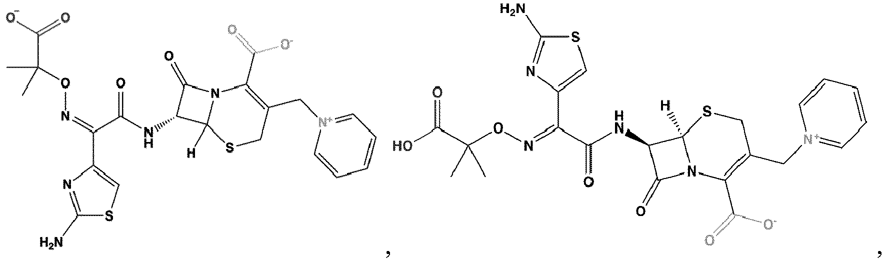 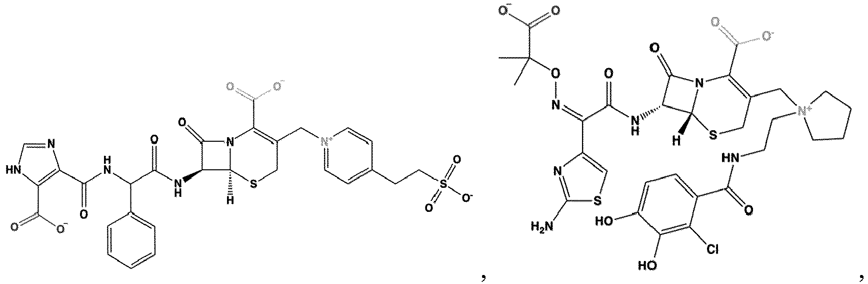 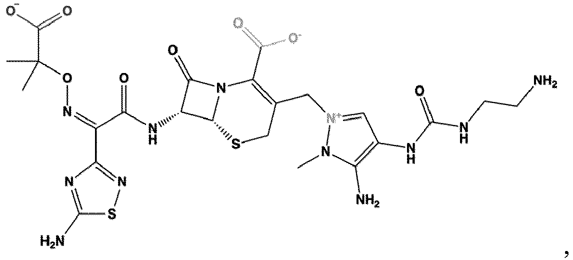 and derivatives thereof, in particular pharmaceutically acceptable salts, solvates, and hydrates thereof.

In one embodiment, said cephalosporin is selected from ceftazidime and derivatives thereof, in particular pharmaceutically acceptable salts, solvates, and hydrates thereof; preferably is ceftazidime pentahydrate.

In one embodiment, said cephalosporin is selected from 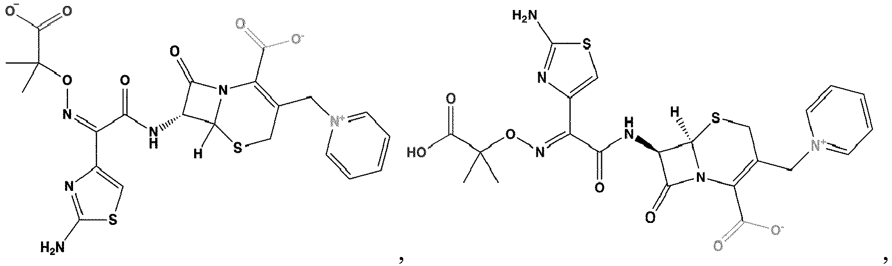 and derivatives thereof, in particular pharmaceutically acceptable salts, solvates, and hydrates thereof.

In one embodiment, said colon cancer is characterized by a resistance to 5-fluorouracil or sensitivity to 5-fluorouracil.

In one embodiment, said colon cancer comprises 5-fluorouracil resistant cancer cells.

In one embodiment, said method involves suppressing cell proliferation and/or inducing apoptosis in cells of said cancer, preferably in 5-fluorouracil resistant cancer cells and/or 5-fluorouracil sensitive cancer cells.

In one embodiment, said method involves suppressing cell proliferation in cells of said cancer by decreasing the metabolic activity of said cells.

In one embodiment, said method involves inducing apoptosis in cells of said cancer by inducing a pre-Gi phase and/or by increasing caspase activity, preferably caspase 3 and/or caspase 7 activity.

In one embodiment, said method involves inducing apoptosis in cells of said cancer by inducing a pre-Gi phase and/or inducing apoptosis by increasing caspase activity, preferably caspase 3 and/or caspase 7 activity.

In one embodiment, in said method, said cephalosporin is administered to a patient in need thereof, preferably a 5-fluorouracil naive patient or a 5-fluorouracil non-responding patient, e.g. a patient suffering from a cancer characterized by 5-fluorouracil resistant cells.

In one embodiment, in said method, said cephalosporin is co-administered with a further drug, preferably further anti-colon cancer drug.

In one embodiment, said further drug is selected from topoisomerase inhibitors, e.g. irinotecan hydrochloride; platinum-based antineoplastic agents, e.g. oxaliplatin; compositions comprising trifluridine and tipiracil hydrochloride; mTOR inhibitors, e.g. everolimus; somatostatin analogues, e.g. lanreotide acetate; anti-CTLA₄ antibodies, e.g. ipilimumab; epidermal growth factor receptor inhibitors, e.g. cetuximab and panitumumab; PD-1 receptor blockers, e.g. nivolumab and pembrolizumab; vascular endothelial growth factor inhibitors, e.g. ramucirumab, bevacizumab, and ziv-aflibercept; and oral multikinase inhibitors, e.g. regorafenib and sorafenib.

In one embodiment, in said method, said cephalosporin is not co-administered with 5-fluorouracil.

In a further aspect, the present invention relates to a pharmaceutical composition for use in a method of preventing and/or treating colon cancer, wherein said composition comprises a cephalosporin, as defined above, and a pharmaceutically acceptable excipient, optionally further comprises a further drug selected from topoisomerase inhibitors, e.g. irinotecan hydrochloride; platinum-based antineoplastic agents, e.g. oxaliplatin; compositions comprising trifluridine and tipiracil hydrochloride; mTOR inhibitors, e.g. everolimus; somatostatin analogues, e.g. lanreotide acetate; anti-CTLA₄ antibodies, e.g. ipilimumab; epidermal growth factor receptor inhibitors, e.g. cetuximab and panitumumab; PD-1 receptor blockers, e.g. nivolumab and pembrolizumab; vascular endothelial growth factor inhibitors, e.g. ramucirumab, bevacizumab, and ziv-aflibercept; and oral multikinase inhibitors, e.g. regorafenib and sorafenib.

In a further aspect, the present invention relates to a method of preventing and/or treating colon cancer, comprising administering an effective amount of a cephalosporin to a patient in need thereof.

In this aspect, the colon cancer, the cephalosporin, the method, and the patient are as defined above.

In a further aspect, the present invention relates to the use of a cephalosporin for the manufacture of a medicament for preventing and/or treating colon cancer.

In this aspect, the cephalosporin and the colon cancer are as defined above.

### DETAILED DESCRIPTION

The invention aims at providing an agent that induces cell death and/or inhibits cell proliferation of 5-fluorouracil resistant and sensitive colon cancer cells. It is a further aim of the invention to provide an agent that is inexpensive and is readily available for treating cancer, preferably colon cancer. Furthermore, it is an aim of the invention to provide a treatment for colon cancer resulting from abnormal cell proliferation. The objects of the invention are solved by a cephalosporin, such as ceftazidime, for use in treating colon cancer cells.

Particularly, the invention provides a cephalosporin antibiotic belonging to the β-lactams family, for use in preventing and/or treating colon cancer, particularly in selective apoptotic cell death-inducing activities and suppression of cell proliferation of human colon cancer cells. The present invention further relates to a cephalosporin, preferably ceftazidime, for use against colon cancer cells, e.g. 5-fluorouracil resistant cancer cells and/or 5-fluorouracil sensitive cancer cells. Furthermore, the invention relates to a cephalosporin for use against colon cancer cells that are sensitive or resistant to 5-fluorouracil. Furthermore, the invention relates to a cephalosporin, which has an anti-cancer effect by inducing apoptotic cell death and/or suppressing colon cancer cell proliferation.

As a repurposed drug with potential anticancer activities, a cephalosporin such as ceftazidime with its well-tolerated side effects, known pharmacokinetics and bioavailability, is a promising new and cheap potential drug. Cephalosporins such as ceftazidime can reach the market in a short period to treat colon cancer. The cephalosporin for use according to the invention, preferably ceftazidime, is highly advantageous in that is well-tolerated, has few side effects, and has a known pharmacokinetic profile. Furthermore, the cephalosporin, e.g. ceftazidime, for use of the invention is highly advantageous in that is low-priced, and thus affordable for emerging market countries and developing countries. Accordingly, the cephalosporin for use according to the invention is advantageous in that it is a cheap and effective anticancer agent. Furthermore, it is advantageous in that is has a known pharmacokinetic profile and high safety. Furthermore, it is advantageous in that it does not have severe side effects. It is also advantageous in that it effectively treats 5-fluorouracil resistant colon cancer cells.

The term "cephalosporin", as used herein, relates to a member of a class of β-lactam antibiotics, e.g. ceftazidime, cefpimizole, cefiderocol, and ceftolozane, particularly ceftazidime. Based on the timeline of drug discovery and their antimicrobial properties, cephalosporins are grouped into different generations, including a third generation and fourth generation. In one embodiment, the cephalosporin for use is a third generation cephalosporin, e.g. ceftazidime or cefpimizole, preferably ceftazidime, or a fourth generation cephalosporin, e.g. cefiderocol or ceftolozane. In one embodiment, the cephalosporin for use is selected from ceftazidime and derivatives thereof, in particular pharmaceutically acceptable salts, solvates, and hydrates thereof, e.g. ceftazidime pentahydrate. In one embodiment, the cephalosporin is ceftazidime pentahydrate. In one embodiment, the cephalosporin is selected from ceftazidime, cefpimizole, cefiderocol, ceftolozane, and pharmaceutically acceptable salts, solvates, and hydrates thereof. In one embodiment, the cephalosporin, e.g. ceftazidime, cefpimizole, cefiderocol, or ceftolozane, is administered in an unprotonated, deprotonated, or protonated form. In one embodiment, when referring to a "cephalosporin", e.g. "ceftazidime", "cefpimizole", "cefiderocol", or "ceftolozane", or a structure thereof, the respective term or structure, respectively, also includes any protonation state thereof, as well as pharmaceutically acceptable salts, solvates, or hydrates thereof. In one embodiment, each of ceftazidime, cefpimizole, cefiderocol, and ceftolozane comprises a nitrogen, preferably positively charged nitrogen, and a first carboxyl group, preferably a negatively charged carboxyl group; wherein the nitrogen and the carboxyl group of said cephalosporin are spaced apart by three carbon atoms. In one embodiment, a cephalosporin comprising a nitrogen, preferably positively charged nitrogen, and a first carboxyl group, preferably a negatively charged carboxyl group, wherein the nitrogen and the carboxyl group of said cephalosporin are spaced apart by three carbon atoms, is selected from ceftazidime, cefpimizole, cefiderocol, ceftolozane, and pharmaceutically acceptable salts, solvates, and hydrates thereof. In a preferred embodiment, a cephalosporin comprising a nitrogen, preferably positively charged nitrogen, and a first carboxyl group, preferably a negatively charged carboxyl group, wherein the nitrogen and the carboxyl group of said cephalosporin are spaced apart by three carbon atoms, is ceftazidime or a pharmaceutically acceptable salt, solvate, or hydrate thereof. In one embodiment, the present invention relates to a cephalosporin for use in a method of preventing and/or treating colon cancer, wherein the cephalosporin is selected from ceftazidime, cefpimizole, cefiderocol, ceftolozane, and derivatives thereof, in particular pharmaceutically acceptable salts, solvates, and hydrates thereof; preferably is ceftazidime or a pharmaceutically acceptable salt, solvate, or hydrate thereof, e.g. ceftazidime pentahydrate.

The term "spaced apart by three carbon atoms", as used herein, relates to a distance of a nitrogen and a first carboxyl group, wherein the nitrogen and the first carboxyl group are connected via three carbon atoms between the nitrogen and the carboxyl group. In one embodiment, the carbon atom of the carboxyl group and the nitrogen have three carbon atoms between them which are connected via covalent bonding. In one embodiment, two of the three carbon atoms connecting the first carboxyl group and the nitrogen are part of a dihydrothiazine ring of the cephalosporin core structure. In one embodiment, the nitrogen and the first carboxyl group are each part of a moiety attached a dihydrothiazine ring of the cephalosporin core structure. For example, the nitrogen is part of a methylpyridinium moiety attached at a C-3 position of the dihydrothiazine ring. For example, the first carboxyl group is attached at a C-2 position of the dihydrothiazine ring. In one embodiment, a cephalosporin comprising a nitrogen, preferably positively charged nitrogen, and a first carboxyl group, preferably a negatively charged carboxyl group, spaced apart by three carbon atoms is selected from ceftazidime, cefpimizole, cefiderocol, and ceftolozane, preferably ceftazidime.

The term "second carboxyl group", as used herein, relates to a carboxyl group different from the first carboxyl group. In one embodiment, the second carboxyl group is not a substituent of the dihydrothiazine ring of the cephalosporin core structure and/or is not part of a moiety attached to the dihydrothiazine ring. In one embodiment, the second, carboxyl group is part of a moiety attached to the β-lactam ring.

The term "third-generation cephalosporin", as used herein, relates to broad-spectrum antimicrobial agents useful in a variety of clinical situations. In one embodiment, a third-generation cephalosporin is selected from the group consisting of ceftazidime, cefpimizole, cefcapene, cefdaloxime, cefdinir, cefditoren, cefetamet, cefixime, cefmenoximem, cefodizime, cefotaxime, cefovecin, cefpodoxime, cefteram, ceftibuten, ceftiofur, ceftiolene, ceftizoxime, ceftriaxone, and cefoperazone; preferably selected from ceftazidime and cefpimizole; more preferably ceftazidime.

The term "fourth-generation cephalosporin", as used herein, relates to the fourth group of cephalosporins discovered. They are structurally related to third-generation cephalosporins but possess an extra ammonium group, particularly a quaternary ammonium group at the C-3' position, allowing for rapid penetration through the outer membrane of Gram-negative bacteria. Fourth-generation cephalosporins typically have a broad-spectrum antimicrobial activity. In one embodiment, a fourth-generation cephalosporin is selected from the group consisting of ceftolozane, cefiderocol, cefclidine, cefepime, cefluprenam, cefoselis, cefozopran, cefpirome, cefquinome, ceftobiprole, and ceftaroline; preferably selected from ceftolozane and cefiderocol.

The term "colon cancer", as used herein, relates to any colon cancer known to the person skilled in the art, e.g., colon cancer characterized by 5-fluorouracil resistant cancer cells or colon cancer characterized by 5-fluorouracil sensitive cancer cells. In one embodiment, said colon cancer is characterized by a resistance to 5-fluorouracil or sensitivity to 5-fluorouracil. In one embodiment, said colon cancer comprises 5-fluorouracil sensitive cancer cells and/or 5-fluorouracil resistant cancer cells. In one embodiment, the cephalosporin for use, preferably ceftazidime for use, suppresses cell proliferation and/or induces apoptosis in cells of said cancer, preferably in 5-fluorouracil resistant cancer cells and/or 5-fluorouracil sensitive cancer cells. In one embodiment, the cephalosporin for use according to the invention is highly advantageous in that it effectively treats 5-fluorouracil resistant cancers and 5-fluorouracil sensitive cancers. In one embodiment, the cephalosporin for use according to the invention is highly advantageous in that it allows for effective treatment of patients suffering from 5-fluorouracil resistant cancers and/or cancers comprising 5-fluorouracil resistant cancer cells. In one embodiment, the cephalosporin for use according to the invention is highly advantageous in that it allows for effective treatment of patients suffering from 5-fluorouracil sensitive cancers and/or cancers comprising 5-fluorouracil sensitive cancer cells. In one embodiment, the cephalosporin for use according to the invention is highly advantageous in that it allows for effective treatment of patients suffering from cancers comprising 5-fluorouracil resistant cancer cells and 5-fluorouracil sensitive cancer cells. In one embodiment, the cephalosporin for use is for treating patients suffering from 5-fluorouracil resistant cancers or 5-fluorouracil sensitive cancers, e.g. 5-fluorouracil resistant cancers. In one embodiment, said colon cancer, preferably colon cancer characterized by 5-fluorouracil resistant cancer cells and/or 5-fluorouracil sensitive cancer cells, is treated by suppressing cell proliferation in cells of said cancer and/or by inducing apoptosis in cells of said cancer using said cephalosporin. In one embodiment, said colon cancer is a cancer characterized by 5-fluorouracil resistant cancer cells, and said colon cancer is treated by the cephalosporin, preferably ceftazidime, inducing apoptosis in 5-fluorouracil resistant cancer cells of said cancer. In one embodiment, said colon cancer is a cancer characterized by 5-fluorouracil sensitive cancer cells, and said colon cancer is treated by the cephalosporin, preferably ceftazidime, suppressing cell proliferation in 5-fluorouracil sensitive cancer cells of said cancer. In one embodiment, a 5-fluorouracil resistant cancer cell is a cancer cell that does not respond to a treatment with 5-fluorouracil. In one embodiment, a 5-fluorouracil sensitive cancer cell is a cancer cell that responds to a treatment with 5-fluorouracil.

The term "ceftazidime", as used herein, relates to a third-generation cephalosporin antibiotic belonging to the β-lactams family. It is typically active against gram-positive and gram-negative aerobic bacteria and resists hydrolysis by most β-lactamases. Ceftazidime is commonly used to treat immunocompromised patients suffering from fever of unknown origin or documented infection. Ceftazidime is well tolerated. Ceftazidime has an established antibacterial activity. In one embodiment, the term "ceftazidime", as used herein, includes ceftazidime pentahydrate, ceftazidime dihydrochloride, ceftazidime sodium, ceftazidime anhydrous, and/or methyl ceftazidime. In one embodiment, ceftazidime is (6R,7R)-7-[[(2Z)-2-(2-amino-1,3-thiazol-4-yl)-2-(2-carboxypropan-2-yloxyimino)acetyl]amino]-8-oxo-3-(pyridin-1-ium-1-ylmethyl)-5-thia-1-azabicyclo[4.2.0]oct-2-ene-2-carboxylate, or a pharmaceutically acceptable salt, solvate, or hydrate thereof. In one embodiment, ceftazidime has a formula C₂₂H₂₂N₆O₇S₂·5H₂O. In one embodiment, ceftazidime has a structure of 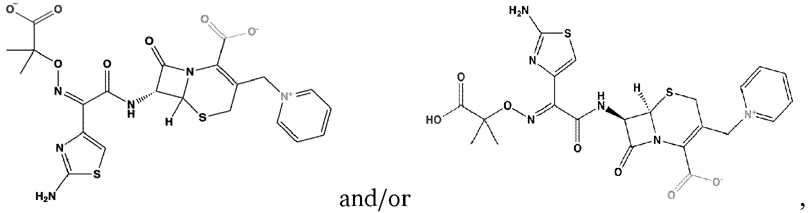 for example depending on its protonation state. In one embodiment, the present invention relates to ceftazidime for use in a method of preventing and/or treating colon cancer. In one embodiment, ceftazidime is in the form of ceftazidime pentahydrate. In one embodiment, ceftazidime is administered in the form of a compound having a structure as shown below 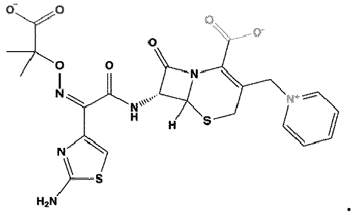 In one embodiment, said cephalosporin, preferably ceftazidime, is administered to a patient in need thereof, wherein said patient is a mammal, preferably a human. In one embodiment, said cephalosporin, preferably ceftazidime, is administered intravenously, intravascularly, orally, intraarticularly, nasally, mucosally, intracolonically, intrabronchially, intrapulmonarily, intrarenally, intrahepatically, intradermally, subcutaneously, intramuscularly, intraocularly, intrathecally, or intranodally.

The term "cefpimizole", as used herein, relates to a third-generation cephalosporin antibiotic. In one embodiment, cefpimizole has a structure of 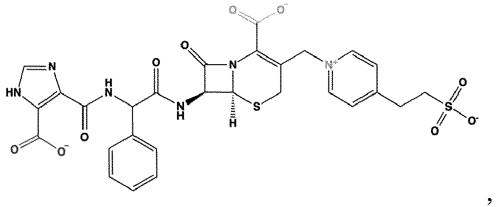 or a pharmaceutically acceptable salt, solvate, or hydrate thereof. In one embodiment, cefpimizole is 2-[1-[[(6R,7R)-2-carboxy-7-[[(2R)-2-[(5-carboxy-1H-imidazole-4-carbonyl)amino]-2-phenylacetyl]amino]-8-oxo-5-thia-1-azabicyclo[4.2.o]oct-2-en-3-yl]methyl]pyridin-1-ium-4-yl]ethanesulfonate, or a pharmaceutically acceptable salt, solvate, or hydrate thereof. In one embodiment, cefpimizole is a third-generation cephalosporin. In one embodiment, the term "cefpimizole", as used herein, includes cefpimizole sodium.

The term "cefiderocol", as used herein, relates to an antibiotic typically used to treat complicated urinary tract infections when no other options are available. In one embodiment, cefiderocol is (6R,7R)-7-[[(2Z)-2-(2-amino-1,3-thiazol-4-yl)-2-(2-carboxypropan-2-yloxyimino)acetyl]amino]-3-[[1-[2-[(2-chloro-3,4-dihydroxybenzoyl)amino]ethyl]pyrrolidin-1-ium-1-yl]methyl]-8-oxo-5-thia-1-azabicyclo[4.2.0]oct-2-ene-2-carboxylate, or a pharmaceutically acceptable salt, solvate, or hydrate thereof. In one embodiment, cefiderocol has a structure of 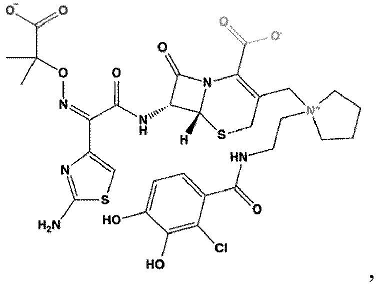 or a pharmaceutically acceptable salt, solvate, or hydrate thereof. In one embodiment, cefiderocol is a fourth-generation cephalosporin. In one embodiment, the term "cefiderocol", as used herein, includes cefiderocol sulfate tosylate.

The term "ceftolozane", as used herein, relates to an antibiotic medication typically used for the treatment of complicated urinary tract infections and complicated intra-abdominal infections in adults. In one embodiment, ceftolozane is (6R,7R)-3-[[3-amino-4-(2-aminoethylcarbamoylamino)-2-methylpyrazol-1-ium-1-yl]methyl]-7-[[(2Z)-2-(5-amino-1,2,4-thiadiazol-3-yl)-2-(2-carboxypropan-2-yloxyimino)acetyl]amino]-8-oxo-5-thia-i-azabicyclo[4.2.0]oct-2-ene-2-carboxylate, or a pharmaceutically acceptable salt, solvate, or hydrate thereof. In one embodiment, ceftolozane has a structure of 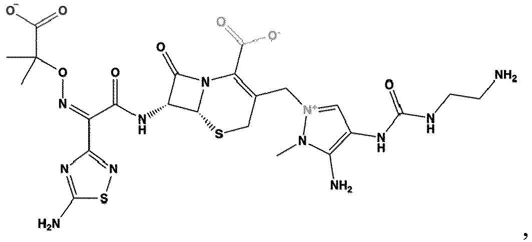 or a pharmaceutically acceptable salt, solvate, or hydrate thereof. In one embodiment, ceftolozane is a fourth-generation cephalosporin. In one embodiment, the term "ceftolozane", as used herein, includes ceftolozane trifluoroacetate salt.

The term "derivatives thereof', as used herein, relates to any variant of a cephalosporin, e.g. any variant of ceftazidime, which maintains the anti-colon cancer activity of the cephalosporin, e.g. ceftazidime, from which it is derived. In one embodiment, a derivative of a cephalosporin is a pharmaceutically acceptable salt, solvate, or hydrate of the cephalosporin. In one embodiment, a derivative, e.g. pharmaceutically acceptable salt, solvate, or hydrate, of ceftazidime is selected from ceftazidime pentahydrate, e.g. C22H₃2N₆O₁₂S₂; ceftazidime dihydrochloride, e.g. C₂₂H₂₄Cl₂N₆O₇S₂; ceftazidime sodium, e.g. C22H₂₁N₆NaO₇S₂; ceftazidime anhydrous, e.g. C₂₂H₂₂N₆O₇S₂; and methyl ceftazidime, e.g. C₂₃H₂₄N₆O₇S₂. In one embodiment, a pharmaceutically acceptable salt of ceftazidime is ceftazidime sodium, e.g. C₂₂H₂₁N₆NaO₇S₂, or ceftazidime anhydrous, e.g. C₂₂H₂₂N₆O₇S₂. In one embodiment, a solvate, preferably pharmaceutically acceptable solvate, of ceftazidime is ceftazidime dihydrochloride, e.g. C₂₂H₂₄Cl₂N₆O₇S₂. In one embodiment, a hydrate, preferably pharmaceutically acceptable hydrate, of ceftazidime is ceftazidime pentahydrate, e.g. C₂₂H₃₂N₆O₁₂S₂. In one embodiment, a derivative, particularly structural derivative, of ceftazidime is methyl ceftazidime, e.g. C₂₃H₂₄N₆O₇S₂. In one embodiment, a derivative, e.g. pharmaceutically acceptable salt, solvate, or hydrate, of cefpimizole is cefpimizole sodium, e.g. C₂₈H₂₅N₆NaO₁₀S₂. In one embodiment, a pharmaceutically acceptable salt of cefpimizole is cefpimizole sodium, e.g. C₂₈H₂₅N₆NaO₁₀S₂. In one embodiment, a derivative, e.g. pharmaceutically acceptable salt, solvate, or hydrate, of ceftolozane is ceftolozane trifluoroacetate salt, e.g. C₂₃H₃₁N₁₂O₈S₂·CF₃COO. In one embodiment, a pharmaceutically acceptable salt of ceftolozane is ceftolozane trifluoroacetate salt, e.g. C₂₃H₃₁N₁₂O₈S₂·CF₃COO. In one embodiment, a derivative, e.g. pharmaceutically acceptable salt, solvate, or hydrate, of cefiderocol is cefiderocol sulfate tosylate, e.g. C₁₁₈H₁₃₈Cl₃N₂₁O₄₇S₁₁. In one embodiment, a hydrate, preferably pharmaceutically acceptable hydrate, of cefiderocol is cefiderocol sulfate tosylate, e.g. C₁₁₈H₁₃₈Cl₃N₂₁O₄₇S₁₁.

In one embodiment, the cephalosporin for use of the invention is selected from ceftazidime, cefpimizole, cefiderocol, ceftolozane, and derivatives thereof; preferably selected from ceftazidime pentahydrate, ceftazidime dihydrochloride, ceftazidime sodium, ceftazidime anhydrous, methyl ceftazidime, cefpimizole sodium, ceftolozane trifluoroacetate salt, and cefiderocol sulfate tosylate; more preferably selected from ceftazidime pentahydrate, ceftazidime dihydrochloride, ceftazidime sodium, ceftazidime anhydrous, and methyl ceftazidime; even more preferably ceftazidime pentahydrate.

In one embodiment, the term "metabolic activity", as used herein, relates to the ability of metabolically active cells to convert tetrazolium salt into a blue formazan product, for example, as measured using an MTT assay. In one embodiment, metabolic activity is measured performing an MTT assay and analyzing absorbance at 540 nm by an ELISA reader.

The term "pre-G1 phase", as used herein, relates to a cell phase in which a cell has a nucleus which is in the process of disintegration and comprises fragmented DNA. In one embodiment, a pre-Gi phase is a phase in which the cell is in the process of dying. In one embodiment, a cephalosporin for use of the invention induces a pre-Gi phase in cancer cells, e.g. 5-fluorouracil resistant cancer cells and/or 5-fluorouracil sensitive cancer cells. In one embodiment, a cephalosporin for use of the invention induces an increase in the number of cancer cells that are in a pre-Gi phase, e.g. an increase in the number of cells that are in a pre-Gi phase in 5-fluorouracil resistant cancer cells and/or 5-fluorouracil sensitive cancer cells.

The term "5-fluorouracil naive patient", as used herein, relates to a patient, which has not been subjected to a treatment with 5-fluorouracil. In one embodiment, a 5-fluorouracil naive patient relates to a patient that has never received a treatment with 5-fluorouracil or has not received a treatment with 5-fluorouracil in at least one year, preferably at least two years; preferably to a patient that has never received a treatment with 5-fluorouracil.

The term "5-fluorouracil non-responding patient", as used herein, relates to a patient that has received a treatment with 5-fluorouracil, but has not shown a therapeutic response to 5-fluorouracil; for example, a therapeutic effect of 5-fluorouracil has not been observed in said patient, such as a decrease in tumor volume and/or tumor growth. In one embodiment, a 5-fluorouracil non-responding patient is a patient suffering from a cancer characterized by 5-fluorouracil resistant cells. In one embodiment, a 5-fluorouracil non-responding patient is a patient suffering from a cancer comprising and/or characterized by 5-fluorouracil resistant cancer cells.

The term "co-administered with a further drug", as used herein, relates to a drug being administered with the cephalosporin for preventing and/or treating colon cancer. For example, such co-administration may relate to a subsequent administration of the cephalosporin and the further drug, or vice versa, or a simultaneous administration of the cephalosporin and the further drug, e.g. in the form of a composition for use comprising the cephalosporin and the further drug. In one embodiment, the further drug is an anti-colon cancer drug, preferably selected from topoisomerase inhibitors, e.g. irinotecan hydrochloride; platinum-based antineoplastic agents, e.g. oxaliplatin; compositions comprising trifluridine and tipiracil hydrochloride; mTOR inhibitors, e.g. everolimus; somatostatin analogues, e.g. lanreotide acetate; anti-CTLA₄ antibodies, e.g. ipilimumab; epidermal growth factor receptor inhibitors, e.g. cetuximab and panitumumab; PD-1 receptor blockers, e.g. nivolumab and pembrolizumab; vascular endothelial growth factor inhibitors, e.g. ramucirumab, bevacizumab, and ziv-aflibercept; and oral multikinase inhibitors, e.g. regorafenib and sorafenib. In one embodiment, the cephalosporin is not co-administered with 5-fluorouracil. In one embodiment, the cephalosporin, particularly ceftazidime, is not co-administered with 5-fluorouracil, since the effect of the cephalosporin and the effect of 5-fluorouracil antagonize each other. In one embodiment, the cephalosporin, particularly ceftazidime, decreases the anticancer effect of 5-fluorouracil; and/or 5-fluorouracil decreases the anticancer effect of the cephalosporin, particularly ceftazidime. In one embodiment, the further drug is not 5-fluorouracil. In one embodiment, a patient that has been subjected to a treatment with 5-fluorouracil, and which does not show a therapeutic response to the treatment with 5-fluorouracil, is subsequently subjected to a treatment with the cephalosporin for use, preferably ceftazidime. In one embodiment, the method comprises administering the cephalosporin for use to a patient, who is a 5-fluorouracil non-responding patient. In one embodiment, the method comprises administering the cephalosporin for use to a patient which has been subjected to a treatment with 5-fluorouracil, but which has not shown a therapeutic response to the treatment with 5-fluorouracil. In one embodiment, the cephalosporin for use in a method of preventing and/or treating colon cancer is administered to a patient suffering from said cancer. In one embodiment, the metabolic system of said patient does not comprise 5-fluorouracil. In one embodiment, 5-fluorouracil is absent in said patient. In one embodiment, 5-fluorouracil is absent in the body of said patient. In one embodiment, 5-fluorouracil is absent in the metabolic system and/or blood stream of said patient. In one embodiment, the cephalosporin for use of the invention is used in the absence of 5-fluorouracil.

In one embodiment, the term "effective amount" of a cephalosporin, e.g. ceftazidime, relates to an amount that delays or stops the progress of the colon cancer and that does not cause an adverse effect that exceeds the benefit of administration. The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

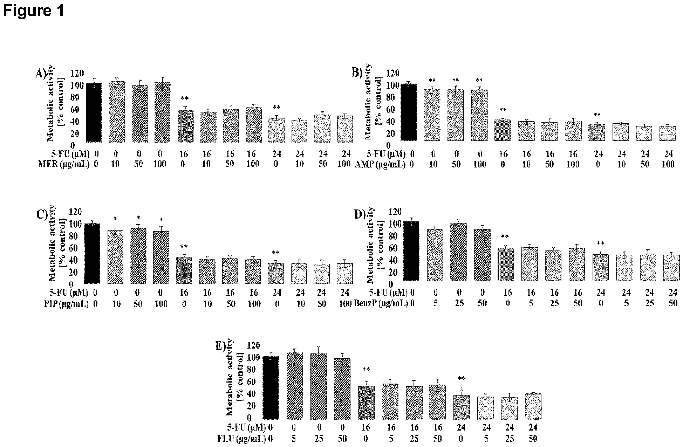

**Figure 1** shows that a combination treatment of 5-fluorouracil (5-FU) and the different antibiotics (ampicillin (AMP), benzylpenicillin (BenP), flucloxacillin (FLU), meropenem (MER) and piperacillin (PIP)) does not influence the inhibiting effect induced by 5-FU. The metabolic activity of HCT-116 cells was measured using MTT assay 48 h post-treatment with 5-FU (16 µM and 24 µM) in the presence and absence of the different antibiotics: MER, AMP and PIP (10, 50 and 100 µg/mL) and BenP and FLU (5, 25 and 50 µg/mL). Results are expressed with respect to their respective control. Each value is the mean ± SEM of three independent experiments. Statistical analysis was performed using one tailed student t-test. *P<0.05 and **P<0.01 significant to control untreated cells.

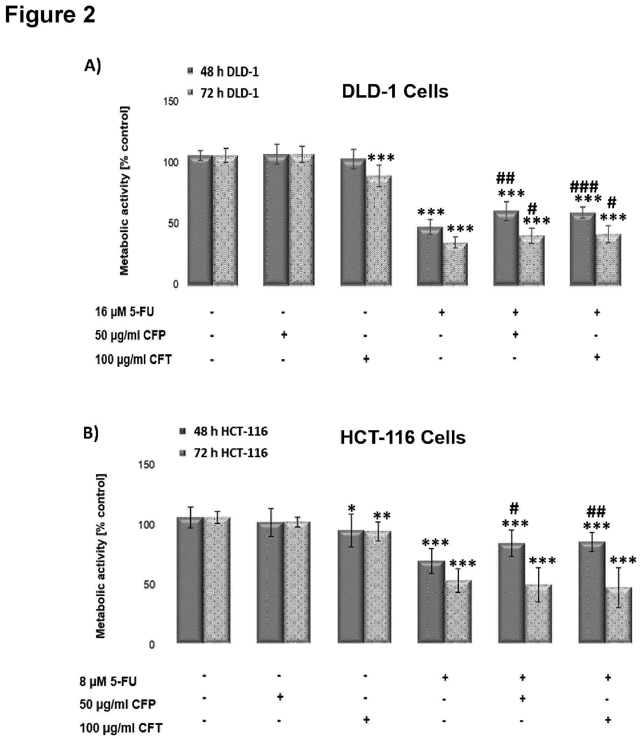

**Figure 2** shows that ceftazidime (CFT) and cefepime (CFP) antagonize 5-fluoroucacil (5-FU)'s effect on two colon cancer cell lines (DLD-1 cells (A) and HCT-116 (B)). Effect of 5-FU (8 µM or 16 µM), CFP (50 µg/mL), CFT (100 µg/mL) and their combinations (5-FU/CFT and 5-FU/CFP) 48 h and 72 h post-treatment on the metabolic activity of DLD-1 and HCT-116 cells was measured using MTT assay. Results are expressed with respect to their respective control. Each value is the mean ± SEM of three independent experiments. Statistical analysis was performed using one tailed student t-test. *P<0.05, P<**0.01, and ***P<0.001 significant to control untreated cells; #P<0.05, ##P<0.01, ###P<0.001 significant with respect to 5-FU.

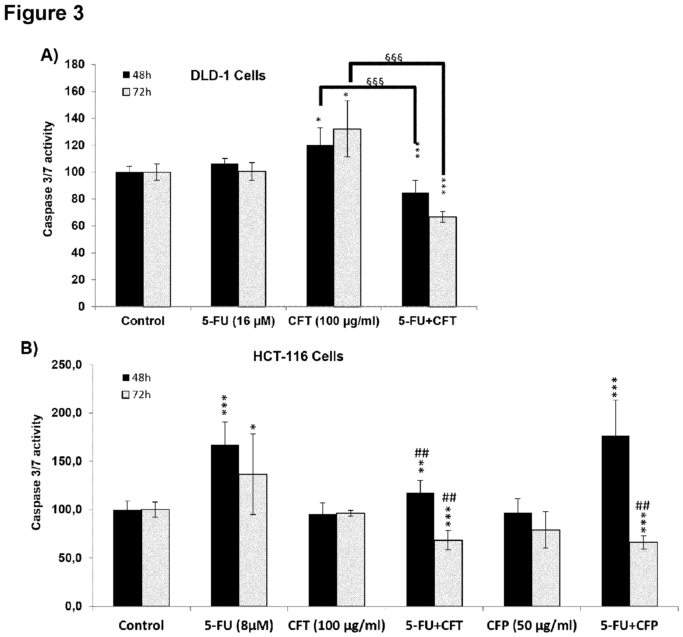

**Figure 3** shows that CFT and 5-FU induce Caspase-3/7 assay in DLD-1 cells (A) and HCT-116 cells (B) respectively. Cells were treated with 5-FU (8 µM for HCT-116 and 16 µM for DLD-1), CFT (100 µg/mL), CFP (50 µg/mL) and their combinations for 48 h and 72 h. Caspase 3/7 activity was measured according to the manufacturer's protocol. Briefly, a preluminescent substrate is cleaved and causes a fluorescent signal proportional to caspase-3/7 activity, which is measured with a microplate reader. Results are expressed with respect to their respective control untreated cells. Standard error of the mean is shown (n = 3). Statistical analysis was performed using one tailed t-test. *P<0.05 and ***P<0.001: significant with respect to control untreated cells; ##P<0.01 and ###P<0.001: significant with respect to 5-FU; ^{§§}P<0.05 and ^{§§§}P<0.001: significant with respect to CFT.

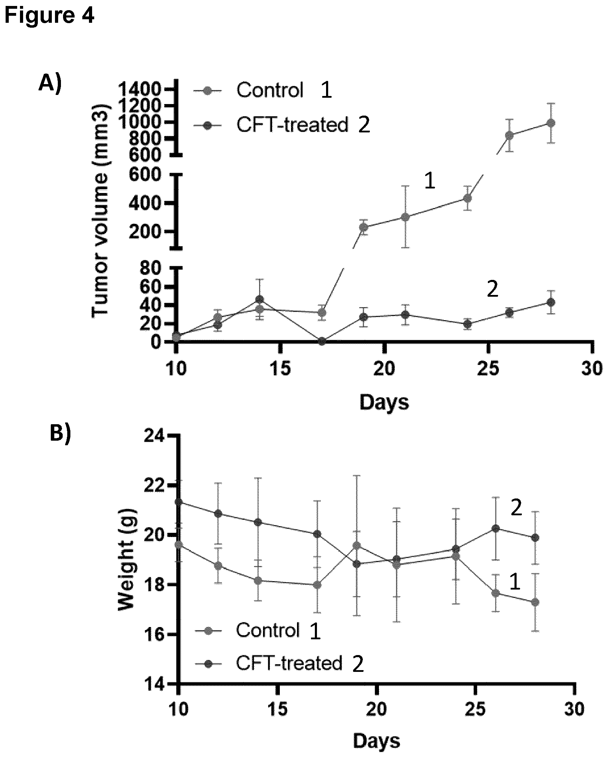

**Figure 4** shows the effect of Ceftazidime on tumor growth (A) and body weight (B) of NOD/SCID mice injected with 3.5 million HCT-116 cells. Tumor measurements were performed three times per week using a sterile Vernier caliper. The volume of the tumor was calculated by the following formula: Volume=π/6*(length × width × height). Each value represents the mean ± SD obtained from six mice from each group. ***p<0.0001, **p<0.001, *p<0.01.

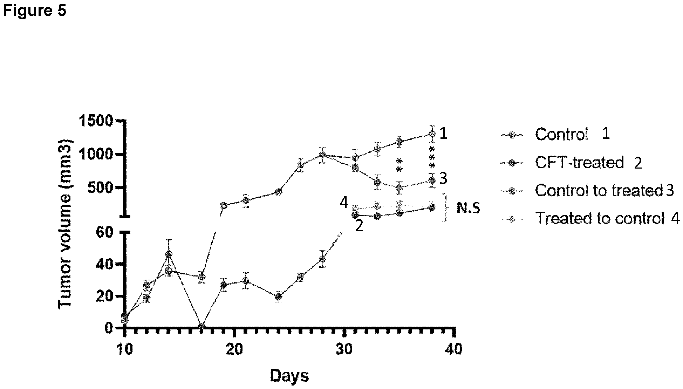

**Figure 5** shows first the effect of ceftazidime on already established tumors and second its post-effect on tumor growth after stopping ceftazidime treatment. To achieve this aim, at week 28, three mice from control untreated-group that have large tumors started their ceftazidime treatment and three mice from ceftazidime-treated group stopped receiving their treatment. Interestingly, in comparison to control untreated mice, which started receiving ceftazidime when the tumor volume was around 990 mm³, a significant inhibition of tumor volume was noted (around 50%), indicating a strong therapeutic effect of ceftazidime. When mice already exposed to ceftazidime for three weeks stopped receiving their treatment, a strong post-tumor inhibitory effect of ceftazidime was seen whereas the tumor did not grow further in the absence of ceftazidime. Accordingly, this data indicates that the cephalosporin, particularly ceftazidime, inhibits tumor growth and suppresses tumors in vivo.

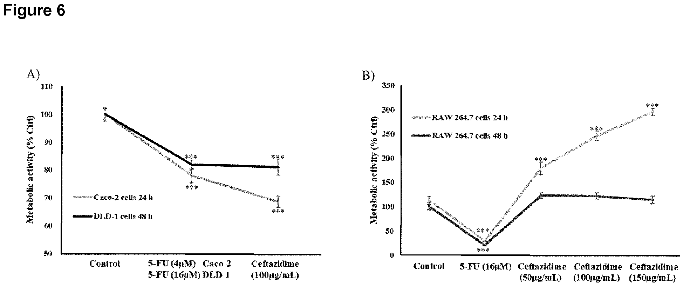

**Figure 6** shows the effect of ceftazidime and 5-fluorouracil (5-FU) on the metabolic activity of DLD-1 and Caco-2 (A) and RAW 264.7 cells (B). MTT assay was used to monitor the metabolic activity of the cells. Each value represents the mean ± SE of three separate experiments done in triplicates.

In the following, reference is made to the examples, which are given to illustrate, not to limit the present invention.

### EXAMPLES

### Example 1:

In this study, the inventors evaluated at the pharmacodynamics (PD) levels, against HCT-116 colon cancer cells, drug-drug interaction (DDI) between 5-fluorouracil (5-FU) and several β-lactams (ampicillin, benzypenicillin, piperacillin, meropenem, flucloxacillin, ceftazidime (CFT), and cefepime (CFP)), widely used in intensive care units. All drugs were tested at clinically achieved concentrations. MTT assay was used to measure the metabolic activity of the cells. Cell cycle profile and apoptosis induction were monitored, in HCT-116 and DLD-1 cells, using propidium iodide staining and Caspase-3/7 activity assay. The uptake of CFT and CFP by the cells was measured using LC-MS/MS method.

The data indicates that despite their limited uptake by the cells, CFT and CFP (two cephalosporins) antagonized significantly 5-FU-induced S-phase arrest (DLD-1 cells) and apoptosis induction (HCT-116 cells). Remarkably, while CFP did not affect the proliferation of colon cancer cells, CFT inhibited the proliferation of DLD-1 cells via apoptosis induction evidenced by an increase in caspase 3/7 activation. Remarkably, the inhibitory effect of CFT is achieved at clinically relevant concentration, which makes it an attractive new potential candidate against colon cancer. Unexpectedly, 5-FU also antagonized CFT's induced cell death in DLD-1 cells.

The study shows that CFP and CFT have adverse effect on 5-FU's action while CFT is a potent anticancer agent that inhibits DLD-1 cells by inducing apoptotic cell death. Further studies are needed to decipher the mechanism(s) responsible for CFT's effects against colon cancer as well as the observed antagonism between CFT, CFP and 5-FU with the ultimate aim of translating the findings to the clinical settings.

### Example 2: Materials and methods

### Cell culture

The experiments were performed using human colon adenocarcinoma cell lines HCT-116 and DLD-1 cells. Both cell lines were cultured in RPMI 1640 (1x)-L-Glutamine medium (Gibco^{™}, Thermo Fisher Scientific, UK) supplemented with 200 mM L-glutamine (PAN-Biotech, Germany) and 10% fetal bovine serum (FBS, PAN-Biotech, Germany). Cells were grown at 37 °C in a humidified atmosphere of 5% CO₂ and 95% air (Heraeus Incubator, Heraeus Instruments GmbH, Germany). For all experiments cells were treated with each of the antibiotics or anticancer agent when alone or in combination, at concentrations that fit within their expected ranges in patients. Cefepime (CFP) was obtained from Rotexmedica (Trittau, Germany) while ceftazidime (CFT), ampicillin (AMP), benzylpenicillin (BenP), flucloxacillin (FLU), meropenem (MER) and piperacillin (PIP) were from Biozol (Eching, Germany). CFP and 5-FU were prepared in water while CFT was dissolved in methanol (VWR ProLabo Chemicals, Germany).

### Metabolic activity assay

MTT assay was used to assess the proliferation rate of HCT-116 and DLD-1 cells. In this assay the ability of metabolically active cells to convert tetrazolium salt into a blue formazan product is measured. The absorption of the dissolved formazan was quantified at 540 nm by an ELISA reader (Bio-Rad iMark Microplate Reader). Briefly, cells were seeded (2.5 x 10⁴ cells/well) in 96-well plates and treated with the tested antibiotics [CFP (50 µg/ml), CFT (100 µg/ml)], and the anticancer agent [5-FU (8 µM or 16 µM)] when alone and in combination for 48 h and 72 h.

### Cell Cycle distribution

Propidium iodide (PI) staining was used to evaluate the effect of the different treatments on the cell cycle. Briefly, DLD-1 and HCT-116 cells were plated at 5×10⁴/ml and treated with CFP (50 µg/ml), CFT (100 µg/ml), 5-FU (8 µM or 16 µM) and their combinations. 48 h and 72 h post-treatment, collected cells were fixed with ice-cold ethanol (70%) and stored at - 20 °C for at least 2 h. Fixed cells, which were washed with PBS and incubated at 37 °C for 1 h with 50 µl RNase (1 mg/ml) (Sigma Aldrich, Germany), were stained for 15 minutes with PI (Molecular Probes, Eugene, Oregon, USA). Cell cycle was monitored using BD FACSDiva^{™} and the percentage of cells in the different phases of the cell cycle (pre-Gi, G0/G1, S, and G2/M) phases was determined using BD FACSDiva^{™} Software.

### Caspase-3/7 activity assay

DLD-1 cells were treated with CFT (100 µg/ml) with and without 5-FU (16 µM) while HCT-116 cells were treated with CFT (100 µg/ml) and CFP (50 µg/ml) with and without 5-FU (8 µM). Caspase-3/7 activity was measured 48 h and 72 h post-treatment according to the manufacturer's protocol (Caspase-Glo-3/7 Assay, Promega Corp, Madison, WI, USA). Briefly, equal volume from treated cells and caspase-3/7 mixture were incubated at room temperature for 3 h and the luminescence was measured by a microplate reader.

### Determination of antibiotics' uptake by the cells

The extracellular and intracellular levels of CFP and CFT were determined using LC-MS/MS method. Briefly, DLD-1 and HCT-116 cells, plated in 12 well plates, were treated with CFP (50 µg/ml), CFT (100 µg/ml) alone and in the presence of 5-FU (8 µM or 16 µM) for 2, 4, 6, and 8 h. The extracellular levels of CFP and CFT were measured in the supernatant. To measure the intracellular levels of CFP and CFT collected cells were lysed by re-suspending each cell pellet in 1 ml of double distilled water followed by three cycles of freeze/thaw in an icebox at -80 °C for 10 min proceeded with 2 min at 37 °C. This step was repeated three times and cell homogenate were vortexed at high-speed prior extraction. 40 µl from each supernatant and cell suspension were combined with 200 µl of internal standard working solution prepared in ice-cold methanol. Samples were then centrifuged and 100 µl of the supernatant were diluted with 400 µl water in glass vials prior analysis with LC-/MS/MS.

### Statistical analysis

Results from at least three independent experiments were summarized and are expressed as means ± standard error of mean (SEM). Statistical significance between different treatments was evaluated using one-tailed Student's *t* -test. The significance level was set to *p* <0.05 for all experiments.

### Example 3: Results

### Ceftazidime and Cefepime antagonize 5-FU's effects on the metabolic activity of DLD-i and HCT-116 cells

The effect of combination treatment of 5-FU and several antibiotics (AMP, BenP, FLU, MER, PIP, CFT, and CFP), widely used in intensive care units (ICU) on the metabolic activity of HCT-116 cells was studied using MTT assay. HCT-116 cells were treated for 48 h and 72 h with clinically relevant concentrations of 5-FU [16 µM (therapeutic concentration), and 24 µM (toxic concentration)] and of the tested antibiotics [highest, middle, and at the lower expected concentration] in patients. The data shows that combination therapy of AMP, FLU, PIP, MER, and BenP with 5-FU does not affect the inhibition induced by the latter one on HCT-116 cells (Figure 1). Surprisingly, 48 h post-treatment CFT and CFP antagonized 5-FU's effect on HCT-116 cells (Figure 2). Unpredictably, a significant decrease in the metabolic activity of DLD-1 cells was observed in response to CFT alone (100 µg/ml) 72 h post-treatment.

### Ceftazidime and Cefepime antagonize 5-FU's effects on the cell cycle profile of HCT-116 and DLD-i cells

To decipher the mechanism(s) of interaction further mechanistic studies were performed using propidium iodide staining to evaluate the effect of the different treatments on the cell cycle profile of the cells. In HCT-116 cells, a time-dependent increase in cell death was a hallmark of 5-FU's effect evidenced by the increase in the percentage of cells in the pre-Gi phase {48 h, 3 fold vs. control, p<0.0001; 72 h, 8.2 fold vs. control, p<0.0001). Up to 72 h post-treatment CFT (100 µg/ml) and CFP (50 µg/ml) alone increased slightly the cells in pre-G1 (9.8% ± 0.9, vs. control, p<0.0001) in HCT-116 cells. Interestingly, 48 h but not 72 h post-treatment CFT (100 µg/ml) and CFP (50 µg/ml) antagonized 5-FU-induced increase in pre-G1 phase (Table 1). This data correlated well with the antagonism shown by the proliferation assay in HCT-116 cells by both cephalosporin's at 48 h but not at 72 h. On the other hand, in DLD-1 cells 5-FU induced a time dependent S-phase arrest (p<0.01; vs. control 48 h and 72 h post-treatment). The findings on 5-FU-induced cell death in both tested cell lines are in accordance with the literature showing that DLD-1 cells are more resistant than HCT-116 cells to 5-FU. CFP and CFT also altered 5-FU-induced changes in the distribution of DLD-1 cells in the different phases of the cell cycle (Table 1). Similar to the proliferation assay CFT alone affected DLD-1 cells significantly whereas it induced a time-dependent accumulation of dead cells in the pre-Gi phase (48 h, 1.9 fold vs. control, p<0.01; 72h, 9.4 fold vs. control, p<0.001). Remarkably, it seems that not only CFT interferes with 5-FU's mechanism of action but also the presence of 5-FU antagonizes CFT-induced cell death in DLD-1 cells, as seen by the decrease in CFT-induced accumulation of cells in pre-Gi phase 72 h post-treatment (Table 1). Collectively, combination of CFT and 5-FU results in an antagonism that affects both drugs. In summary, CFT alone, which significantly inhibited DLD-1 cells, did not significantly affect HCT-116 cells. CFP, on the other hand, had no significant effect on both cell lines but antagonized 5-FU's effect although to a lesser extent in comparison to CFT.

**Table 1: CFT and CFP antagonize 5-FU-induced increase in the percentage of cells in pre-G1 phase. Propidium iodide staining was used to monitor the cell cycle of HCT-116 cells (A) and DLD-1 cells (B) 48 h and 72 h post-treatment with CFT (100 µg/mL), CFP (50 µg/mL), 5-FU (8 µM for HCT-116 and 16 µM for DLD-1) and their combinations. The distribution of the cells in pre-G1, G0/G1, S, and G2/M phases was determined using FACScan flow cytometry. Their percentages were determined and shown as the mean ± SE of three independent experiments done in duplicate. Statistical analysis was performed using one tailed student t-test. ^{a}P<0.05 significant to control untreated cells; ^{b}P<0.05 significant with respect to 5-FU; ^{c}P<0.05 significant with respect to CFT or CFP.**

| **A) HCT-116** | | **0** | | **5-FU (8 µM)** | |
|---|---|---|---|---|---|
| | | ***48 h*** | ***72 h*** | ***48 h*** | ***72 h*** |
| **0** | ***Pre G1*** | 4.2 ± 0.7 | 4.0 ± 0.4 | 16.9 ± 2.5^{(a)} | 36.7 ± 11.7^{(a)} |
| | ***G0*/*G1*** | 55.9 ± 4.5 | 62.7 ± 2.0 | 48.4 ± 2.3^{(a)} | 39.0 ± 9.5^{(a)} |
| | ***S*** | 11.4 ± 1.7 | 6.6 ± 1.6 | 10.6 ± 1.0 | 6.6 ± 0.7 |
| | ***G2*/*M*** | 23.4 ± 2.5 | 21.0 ± 1.4 | 22.3 ± 2.9 | 16.4 ± 2.4^{(a)} |
| **CFP** | ***Pre G1*** | 4.9 ± 1.6 | 9.3 ± 2.9^{(a)} | 12.6 ± 1.6 ^{(a,b,c)} | 23.2 ± 5-3^{(a,c)} |
| **50 µg/ml** | ***G0*/*G1*** | 51.7 ± 2.8 | 53.8 ± 3.6^{(a)} | 53.9 ± 3.9 | 51.4 ± 4.1^{(a)} |
| | ***S*** | 13.6 ± 1.6 | 7.4 ± 1.4 | 9.7 ± 0.9^{(c)} | 6.4 ± 0.6 |
| | ***G2*/*M*** | 24.6 ± 0.9 | 23.6 ± 2.2^{(a)} | 22.5 ± 2.9 | 17.5 ± 2.3^{(a,c)} |
| **CFT** | ***Pre G1*** | 6.4 ± 2.3^{(a)} | 9.8 ± 0.9^{(a)} | 8.9 ± 1.6^{(a,b)} | 35.6 ± 6.4 ^{(a,c)} |
| **100 µg/ml** | ***G0*/*G1*** | 53-3 ± 1.7 | 56.9 ± 1.3^{(a)} | 53.6 ± 3.3^{(b)} | 41.6 ± 2.6 ^{(a,c)} |
| | ***S*** | 12.8 ± 2.9 | 6.6 ± 1.0 | 10.8 ± 1.0 | 8.0 ± 0.7^{(b)(a,c)} |
| | ***G2*/*M*** | 24.4 ± 1.2 | 24.3 ± 0.5^{(a)} | 25.7 ± 5.3 | 14-3 ± 4.3 |

| **B) DLD-1** | | **0** | | **5-FU (16 µM)** | |
|---|---|---|---|---|---|
| | | ***48h*** | ***72 h*** | **48 *h*** | ***72h*** |
| **0** | ***Pre Gi*** | 3.2 ± 1.6 | 3.8 ± 1.1 | 6.3 ± 2.3 | 4.7 ± 0.3 |
| | ***G0*/*G1*** | 53.4 ± 2.5 | 68.0 ± 2.0 | 49.3 ± 4.4 | 69.4 ± 5.0 |
| | ***S*** | 16.4 ± 2.6 | 8.6 ± 0.9 | 32.0 ± 6.1^{(a)} | 20.1 ± 2.9^{(a)} |
| | ***G2*/*M*** | 22.4 ± 2.4 | 19.1 ± 1.9 | 10.3 ± 1.7^{(a)} | 5.4 ± 2.7^{(a)} |
| **CFP** | ***Pre G1*** | 2.6 ± 0.3 | 9.5 ± 4.1^{(a)} | 4.0 ± 1.2^{(c)} | 13.6 ± 1.3^{(a,b)} |
| **50 µg/ml** | ***G0*/*G1*** | 48.8 ± 4.0 | 59.6 ± 3.6^{(a)} | 51.4 ± 9.0 | 64.6 ± 3.7 |
| | ***S*** | 29.2 ± 8.1 | 13.1 ± 3.3^{(a)} | 31-7 ± 5.3 | 17.6 ± 1.2 ^{(a)} |
| | ***G2*/*M*** | 18.4 ± 4.3 | 17.0 ± 3.5 | 12.8 ± 5.0 | 4.3 ± 2.7^{(a,c)} |
| **CFT** | ***Pre G1*** | 9.3 ± 2.5^{(a)} | 40.6 ± 5.2^{(a)} | 7.1 ± 1.5 | 21.0 ± 6.0^{(a,b,c)} |
| **100 µg/ml** | ***G0*/*G1*** | 52.6 ± 3.3 | 38.2 ± 4.3^{(a)} | 40.4 ± 3.0^{(a,b,c)} | 54.6 ± 7.1^{(a,b,c)} |
| | ***S*** | 14.6 ± 3.2 | 8.4 ± 0.6 | 30.3 ± 7.5^{(a,c)} | 10.1 ± 1.3^{(b)} |
| | ***G2*/*M*** | 15-7 ± 2.8^{(a)} | 12.0 ± 1.6^{(a)} | 18.2 ± 3.2^{(b)} | 13.7 ± 1.0^{(a,b)} |

### Combination treatment antagonize apoptosis induction by CFT in DLD-1 and 5-FU in HCT-116 cells

To confirm CFT-induced cell death and the antagonism exerted by 5-FU on DLD-1 cells, the activity of caspase 3/7 was measured 48 h and 72 h following treatment with CFT alone, 5-FU alone, and their combination. As seen in Figure 3, CFT alone increased in time-dependent manner the activity of caspase 3/7 {20% (48 h, *p*<0.05), 32.1% (72 h, *p*<0.05)}, indicating that CFT-induces apoptosis in DLD-1 cells. The increase in caspase 3/7 correlates well with CFT-induced increase in pre-Gi phase of the cell cycle (Table 1). 5-FU alone did not induce caspase 3/7 activity confirming further that in DLD-1 cells and up to 72 h 5-FU induced S-phase arrest. As seen in the cell cycle analysis, combination of CFT with 5-FU abolished significantly (p<0.001) apoptosis induction by CFT 48 h and 72 h post-treatment (Figure 3A). Likewise, CFT antagonized 5-FU-induced apoptosis in HCT-116 cells 48 and 72h post treatment CFP's effect was seen only 72h post-treatment (Figure 3B).

### CFT and CFP antagonize 5-FU's effect without entering the cells

Conflicting data exists regarding the ability of cephalosporin's to cross the plasma membrane of the cells. The fact that significant antagonism was observed between 5-FU and both antibiotics (CFT and CFP), the inventors opted for evaluating whether CFT and CFP are taken up by the cells or whether they induce their effects by residing in the extracellular milieu. The potential uptake of CFT and CFP in the presence and absence of 5-FU was evaluated in DLD-1 and HCT-116 cells up to 8 h post-treatment. HCT-116 and DLD-1 cells were treated with CFT (100 µg/ml) and CFP (50 µg/ml) alone and in combination with either 8 µM or 16 µM 5-FU depending on the cell line. The cells and supernatants were collected up to 8 h post-treatment and processed for analysis by LC-MS/MS. The detected concentration of CFT in the supernatant of DLD-1 and HCT-116 treated cells over 8 h did not decrease significantly (Table 2). Even though up to 8 h of treatment the uptake of CFT by both cell lines is negligible, it is almost 2.5 fold higher in HCT-116 cells (0.047-0.066%) compared to DLD-1 cells (0.019-0.025%). Remarkably, in the presence of 5-FU the uptake of CFT was significantly decreased after 2 h and 4 h of incubation by 35% (P=0.004) and 50% (P=0.038), respectively in HCT-116 cells while in DLD-1 cells the decrease was only observed at 2 h post-treatment (*P*=0.032). Remarkably, 8 h post-treatment a 35% increase in the uptake of CFT (*P*<0.002) was observed in DLD-1 cells; however, the increased trend measured in HCT-116 cells did not reach significance.

**Table 2: The uptake of CFT in HCT-116 cells is 2.5 fold higher than in DLD-1 cells. In the presence of 5-FU CFT's uptake was significantly decreased in HCT-116 cells an effect that is less pronounced in DLD-1 cells. Concentrations of CFT when alone or combined with 5-FU, in cell lysates and supernatants (DLD-1 and HCT-116 cells) up to 8h after treatment were measured by LC-MS/MS. A) CFT (100 µg/ml) ± 5-FU (8 µM) in HCT-116 cells. B) CFT (100 µg/ml) ± 5-FU (16 µM) in DLD-1 cells. p<0.05 (CFT alone vs. in combination), n = 3.**

| **A)** | **DLD-1 cells** | **CFP alone (50 µg/ml)** | **CFP (50 µg/ml) + 5-FU (16 µM)** | **P value alone vs. combination** |
|---|---|---|---|---|
| **2 h** | Supernatant | 50.000 ± 1.697 | 49.261 ± 7.212 | 0.440 |
| | Lysate | 0.012 ± 0.005 | 0.027 ± 0.006 | 0.059 |
| **4 h** | Supernatant | 54.187 ± 0.849 | 51.847 ± 4.879 | 0.251 |
| | Lysate | 0.010 ± 0.0002 | 0.006 ± 0.006 | 0.192 |
| **6 h** | Supernatant | 45.813 ± 3.818 | 60.714 ± 9.970 | 0.069 |
| | Lysate | 0.014 ± 0.0001 | 0.014 ± 0.001 | 0.138 |
| **8 h** | Supernatant | 47.947 ± 12.644 | 50.082 ± 4.371 | 0.377 |
| | Lysate | 0.012 ± 0.001 | 0.015 ± 0.013 | 0.376 |
| | | | | |

| **B)** | **HCT-116 cells** | **CFP alone (50 µg/ml)** | **CFP (50 µg/ml) + 5-FU (8 µM)** | **P value alone vs. combination** |
|---|---|---|---|---|
| **2 h** | Supernatant | 44.100 ± 3.395 | 42.900 ± 5.515 | 0.409 |
| | Lysate | 0.018 ± 0.004 | 0.016 ± 0.001 | 0.305 |
| **4 h** | Supernatant | 34.500 ± 3.818 | 27.885 ± 2.440 | 0.087 |
| | Lysate | 0.022 ± 0.005 | 0.016 ± 0.009 | 0.242 |
| **6 h** | Supernatant | 35.250 ± 1.485 | 34.400 ± 3.516 | 0.388 |
| | **Lysate** | **0.050** ± **0.003** | **0.026** ± **0.001** | **0.005** |
| **8 h** | Supernatant | 35.850 ± 1.909 | 41.000 ± 5.679 | 0.161 |
| | **Lysate** | **0.032** ± **0.002** | **0.007 ± 0.001** | **0.002** |

**Table 3: The uptake of CFP in HCT-116 cells is higher than in DLD-1 cells. CFP concentration in combination treatment with 5-FU was not different to single treatment in DLD-1 cells but was inhibited in HCT-116 cells. Concentrations of CFP when alone or combined with 5-FU, in cell lysates and supernatants (DLD-1 and HCT-116 cells) up to 8 h after treatment were measured by LC-MS/MS. A) CFP (50 µg/ml) ± 5-FU (8 µM) in HCT-116 cells. B) CFP (50 µg/ml) ± 5-FU (16 µM) in DLD1 cells. p<0.05 (CFP alone vs. in combination), n = 3.**

| **A)** | **DLD-1 cells** | **CFT alone (100 µg/ml)** | **CFT (100 µg/ml) + 5-FU (16 µM)** | **P value alone vs. combination** |
|---|---|---|---|---|
| **2 h** | Supernatant | 100.000 ± 2.970 | 97.736 ± 6.804 | 0.311 |
| | Lysate | **0.024** ± **0.003** | **0.016** ± **0.0003** | **0.032** |
| **4 h** | Supernatant | 97.073 ± 5.728 | 98.205 ± 2.551 | 0.356 |
| | Lysate | 0.026 ± **0.002** | 0.024 ± 0.007 | 0.428 |
| **6 h** | Supernatant | 91.335 ± 7.637 | 108.665 ± 22.650 | 0.145 |
| | Lysate | 0.033 ± **0.003** | 0.034 ± 0.004 | 0.415 |
| **8 h** | Supernatant | 98.126 ± 5.940 | 95.628 ± 5.881 | 0.297 |
| | Lysate | **0.032** ± **0.001** | **0.049** ± **0.001** | **0.002** |

| **B)** | **HCT-116 cells** | **CFT alone (100 µg/ml)** | **CFT (100 µg/ml) + 5-FU (8 µM)** | **P value alone vs. combination** |
|---|---|---|---|---|
| **2 h** | Supernatant | 96.900 ± 3.818 | 93.450 ± 0.636 | 0.167 |
| | Lysate | **0.047 ± 0.003** | **0.032 ± 0.0002** | **0.004** |
| **4 h** | Supernatant | 105.000 ± 4.168 | 97.100 ± 12.061 | 0.172 |
| | Lysate | **0.066 ± 0.012** | **0.033 ± 0.006** | **0.038** |
| **6 h** | Supernatant | 96.750 ± 8.697 | 95.300 ± 11.915 | 0.447 |
| | Lysate | 0.056 ± 0.001 | 0.068 ± 0.012 | 0.139 |
| **8 h** | Supernatant | 88.900 ± 8.326 | 86.850 ± 4.031 | 0.388 |
| | Lysate | 0.053 ± 0.010 | 0.108 ± 0.027 | 0.055 |

Interestingly, in DLD-1 cells, the levels of CFP, when alone or combined with 5-FU did not change in both the supernatant and lysate over 8 h of incubation. On the other hand, in HCT-116 cells, a significant decrease in the levels of CFP in the supernatant was observed (44.1 to 35.9, *p*<0.05) along with a slight increase in its levels in cell lysates at 6 h and 8 h of treatments. Yet, the increase in cell lysate does not add up for the loss observed in the supernatant, leading to the assumption that in HCT-116 cells there is non-specific or specific binding of CFP to plasma membrane. This is further supported by the fact that the level of CFP alone or in combination with 5-FU is stable in media alone (Table 4). Interestingly, the uptake of CFP by HCT-116 cells is inhibited significantly in the presence of 5-FU at 6 h and 8 h of incubation. Nonetheless, it seems that in HCT-116 cells the cell entry of CFP, which is inhibited significantly in the presence of 5-FU at 6 h and 8 h of incubation, is three fold higher than in DLD-1 cells 6 h and 8 h post-treatment.

**Table 4: Stability of CFT and CFP when alone or combined with 5-FU incubated up to 8 h in media as measured by LC-MS/MS. A) CFP (50 µg/ml) ± 5-FU (8 µM and 16 µM). B) CFT (100 µg/ml) ± 5-FU (8 µM and 16 µM). P value (a) (between antibiotic alone and 5-FU (8 µM)); P value (b) (between antibiotic alone and 5-FU (16 µM)), n = 3.**

| **A) Stability test** | **CFP (50 µg/mL)** | **CFP (50 µg/mL) + 5-FU (8 µM)** | **CFP (50 µg/mL) + 5-FU (16 µM)** | **P value (a)** | **P value (b)** |
|---|---|---|---|---|---|
| **2h** | 49.9 ± 9.5 | 45.3 ± 6.4 | 50.9 ± o.6 | 0.3 | 0.5 |
| **8h** | 48.8 ± 5.3 | 50.9 ± 7.0 | 47.2 ± 5.9 | 0.4 | 0.4 |

| **B) Stability test** | **CFT (100 µg/mL)** | **CFT (100 µg/mL) + 5-FU(8 µM)** | **CFT (100 µg/mL) + 5-FU (16 µM)** | **P value (a)** | **P value (b)** |
|---|---|---|---|---|---|
| **2h** | 104.4 ± 2.5 | 112.1 ± 4.4 | 94.6 ± 7.5 | 0.1 | 0.1 |
| **8h** | 103.4 ± 10.8 | 110.8 ± 11.3 | 103.4 ± 11.7 | 0.2 | 0.3 |

### Example 4: Discussion

Albeit the improvement in the prediction of DDI over the past years, unexpected drug-drug interaction (DDI) occur. DDI can lead to poor clinical outcome via the involvement of unknown or uncommon metabolic pathways. Unfortunately, it is estimated that 4% of death in cancer patients is due to DDI. Considering the increase in the awareness about toxicities relating to concomitant presence of anti-cancer drugs and antibiotics, the number of reported data is still insufficient. Most studies report on the increase in the toxicity, due to interactions at the PK level, of methotrexate (MTX), an anti-folate agent effective against different types of tumors, when in combination with different classes of antibiotics such as fluoroquinolones, β-lactams (i.e.: penicillin and cephalosporin), and sulfonamide. Interaction at the PD level has been shown where an enhancement of MTX' anti-folate effect was observed in the presence of Trimethoprim, an antibiotic agent with anti-folate property. Unfortunately, data on patients treated with other anti-cancer compounds are scarce.

This study shows that among the seven tested antibiotics, CFT and CFP (two cephalosporins that belong to the β-lactams class of antibiotics) antagonized significantly 5-FU-induced cell death in HCT-116 and DLD-1 cells (Figures 2 and 3). This data indicates that both antibiotics reverse 5-FU's effect in two different colon cancer cell lines regardless of its mechanism of action (S-phase arrest vs. apoptosis induction) (Table 1). Interestingly, the reported antagonism has not been shown previously.

Many approved drugs exhibit off target activities that might be considered beneficial. The fact that the safety and formulation of approved drugs are already established their transition and use against the new targets is facilitated. Another exciting finding from this study is the potent inhibitory effect of CFT against DLD-1 cells, observed at clinically relevant concentration. CFT-induced its effect through the induction of apoptosis as evidenced by the increase in caspase-3/7 activity (Figure 3). This data provide CFT as a potential new drug active against colon cancer cells. Surprisingly, not only CFT antagonized 5-FU's effect but also 5-FU antagonized CFT-induced apoptosis in DLD-1 cells (Figure 3). HCT-116 cells, on the other hand, were less sensitive to CFT, the reason for which remains to be elucidated. Not all β-lactams have inhibitory activities against cancer as seen in this study that shows an anticancer activity of CFT but not the structurally similar antibiotic CFP. In fact, those cephalosporins that show an anticancer effect comprise a nitrogen, preferably positively charged nitrogen, and a first carboxyl group, preferably a negatively charged carboxyl group; wherein the nitrogen and the carboxyl group of said cephalosporin are spaced apart by three carbon atoms, e.g. ceftazidime, cefpimizole, cefiderocol, and ceftolozane.

Despite the potential uptake of cephalosporins by the cells, this is still controversial due to their low lipophilicity. 5-FU, on the other hand, is a small molecule that enters the cells and through the activation of several different cascades leads to cell death. Consequently, to have a better insight about the mechanism of interaction the inventors studied the uptake of CFP and an exemplary cephalosporin, namely CFT, by the cells and evaluated whether they are able to cross the plasma membrane of colon cancer cells. LC-MS/MS was used to measure the levels of CFP and CFT inside the cells and in the supernatants up to 8h of treatment when alone as well as in combination with 5-FU. The data shows that CFP and CFT mainly reside in the extracellular milieu while their minimal uptake differ between the tested cell lines. It is worth mentioning that the similar average uptake of CFP and CFT by the cells (around 0.02%) in DLD-1 cells is less than in HCT-116 cells (2 folds higher). Nonetheless, it seems that in HCT-116 cells, the cell entry of CFP is significantly inhibited in the presence of 5-FU at 6 h and 8 h of incubation through an unknown mechanism that needs further investigation. Collectively, the data implies that CFP and CFT exert their antagonism through the activation of intracellular cascades counteracting those induced by 5-FU.

This study reveals data about adverse effects that could result from the combination of the widely used antineoplastic agent 5-FU and two cephalosporins (CFP and CFT). The lack of information regarding potential DDI between 5-FU, CFP, and CFT emphasizes the importance of the current findings. The potent inhibitory effect through apoptosis induction of CFT in DLD-1 cells along the fact that these cells are considered moderately resistant to 5-FU emphasize the importance of CFT a s new candidate to treat colon cancer.

### Example 5: In vivo analysis

### Xenograft model of colon cancer cells

Twelve NOD/SCID mice were randomly distributed into two groups (6 mice/group). HCT-116 colon cancer cells (3.5 million) suspended in sterile physiological solution (0.9% NaCl) were injected subcutaneously into the flank of 4 to 6-week old mice. Animals were kept in a light- and temperature-controlled environment and provided with food and water ad libitum. The tumor size was monitored and determined three times per week using a sterile Vernier caliper. Tumor volume was calculated by the following formula: Volume=π/6^{∗}(length × width × height). Once the tumor size reached seven mm³, mice were treated three times per week for 21 days with intraperitoneal injections of vehicle (control group) or ceftazidime (10 mg/mouse). Growth delay was assessed by comparing the size of tumors in treated animals to those of control animals. Ethical approval was obtained before the beginning of the experiments.

Herein shown is an anticancer activity of ceftazidime in vivo in a xenograft model of colon cancer cells. Mice from control and treated groups have been inoculated with HCT-116 cells and monitored for tumor growth. Once the tumor size reached around seven mm³, mice from the treated group received intraperitoneal injection of Ceftazidime (10mg/mouse) three times per week. Control mice received the vehicle only. The tumor size on day 1 of treatment did not differ significantly (p=0.12) between both groups. Three weeks post-treatment, the volume of xenografts of HCT-116 cells reached 998.5 mm³ in the control group, while in the Ceftazidime-treated group, the tumor size was 45.3 mm³, confirming a 95% inhibition (p<0.00001) (Figure 4A).

Furthermore, the average weight of the mice in control and Ceftazidime-treated groups on the day of the first Ceftazidime treatment were 19.2 g and 21.3 g, respectively. After three weeks, their body weights were 17.3 g (control group) and 19.8 g (Ceftazidime group) (Figure 4B). This data indicates that control animals lost 11.7% of their weight. In comparison, Ceftazidime treated mice lost only 6.8%, which shows that Ceftazidime has a significant protective effect of 58% (p=0.0001). In addition, none of the animals died in both treated and non-treated groups confirming that Ceftazidime is well-tolerated. This demonstrates that Ceftazidime is an efficient agent for inhibiting tumor growth in vivo. Colon cancer can be treated via administering an adequate amount of Ceftazidime to a target who is in need of treatment. According to the present invention, a suitable amount of Ceftazidime is an amount that delays or stops tumor growth, and that does not cause an adverse effect that exceeds the benefit of the administration.

### Example 6: Ceftazidime reduces the metabolic activity of colon cancer cells and induces apoptosis

### Cells and cell culture

The cell lines used for the in vitro studies include: DLD-1 (human colorectal cancer, resistant to conventional therapy with 5-Fluorouracil), Caco-2 (human colorectal cancer, sensitive to conventional therapy with 5-Fluorouracil), and Raw 264.7 (murine macrophages). DLD-1 cells were grown in RPMI 1640 (1x)-L-Glutamine medium (GipcoTM, Thermo Fisher Scientific, UK) containing 10% fetal bovine serum (FBS) (PAN-Biotech, Germany) and 1% L-glutamine (PAN-Biotech). Caco-2 cells were grown in DMEM (Sigma Aldrich, Germany) with 10% FBS and 1% non-essential amino acids (GipcoTM, Thermo Fisher Scientific, UK). RAW 264.7 cells were grown in DMEM low glucose (Sigma Aldrich, Germany) supplemented with 10% FBS. Cells were grown at 37 °C in a humidified atmosphere of 5% CO2, 95 % air.

### Treatment

Ceftazidime, obtained from Dr. Friedrich Eberth Arzneimittel GmbH, Germany, was prepared at (5mg/mL) in water (VWR ProLabo Chemicals, USA). Ceftazidime stock was aliquoted and stored at -80 °C. On the day of treatment, a fresh aliquot of Ceftazidime was thawed at room temperature and added to cells at concentrations expected in patients following standard antimicrobial therapy. 5-Fluorouracil (Accord Healthcare GmbH, Germany) was used a positive control at 16µM for DLD-1 cells and 4µM for Caco-2 cells. In all experiments, cells were treated at 40-60 % confluency.

### Metabolic activity of the cells

Cells were plated at 2^{∗}104cells/ml in 96-well plates and incubated overnight. The following day, cells were treated in triplicate with Ceftazdime or 5-fluorouracil for 24-48 h, depending on the cell line used. Metabolic activity of the cells was studied using the MTT assay that measures the ability of metabolically active cells to convert tetrazolium salt into a blue formazan product, the absorbance of which is recorded at 540 nm by an ELISA reader (Bio-Rad iMark Microplate Reader).

### Cell Cycle distribution

Propidium iodide (PI) staining was used to evaluate the effect of the different treatments (Ceftazidime (100 µg/ml) or 5-Fluorouracil (16 µM)) on the cell cycle profile of DLD-1 cells. Briefly, DLD-1 cells were plated at 5^{∗}104/ml and treated for 48 h and 72 h. Collected cells were fixed with ice-cold ethanol (70%) and stored at -20°C for at least 2 h. Fixed cells, which were washed with PBS and incubated at 37°C for 1 h with 50 µl RNase (1 mg/ml) (Sigma Aldrich, Germany), were stained for 15 minutes with PI (Molecular Probes, Eugene, Oregon, USA). Cell cycle was monitored using BD FACSDiva^{™} and the percentage of cells in the different phases of the cell cycle (pre-Gi, G0/G1, S, and G2/M) phases was determined using BD FACSDiva^{™} Software.

### Caspase-3/7 activity assay

DLD-1 cells were treated with CFT (100 µg/ml) or 5-Fluorouracil (16 µM). Caspase-3/7 activity was measured 48 h and 72 h post-treatment according to the manufacturer's protocol (Caspase-Glo-3/7 Assay, Promega Corp, Madison, WI, USA). Briefly, equal volume from treated cells and caspase 3/7 mixture were incubated at room temperature for 3 h and the luminescence was measured by a microplate reader.

### Result

Ceftazidime was assayed in vitro against two colon cancer cell lines DLD-1 and Caco-2, respectively resistant and sensitive to 5-Fluorouracil. With regard to the effect on the metabolic activity of the cells, ceftazidime is more potent than 5-Fluorouracil against Caco-2 while its effect on DLD-1 is comparable to 5-Fluorouracil. Using propidium iodide staining it was found that ceftazidime increases the percent of cells in the pre-Gi phase, an effect that was attributed to apoptosis induction evidenced by an increase in Caspase-3/7 activity. Ceftazidime is more potent than 5-fluorouracil that arrests DLD-1 cells in the S-phase of the cell cycle but is unable to induce apoptosis in this resistant cell line. Furthermore, in opposition to the strong toxicity/inhibitory effect associated with 5-fluorouracil against macrophages (RAW 264.7), ceftazidime increased the metabolic activity of the cells only in the first 24 h, which was then restored back to control at 48 h. This data proposes ceftazidime as a more potent new anticancer drug that exhibits growth inhibitory effects on Caco-2 cells, which are sensitive to 5-fluorouracil, and induces apoptosis in DLD-1 cancer cells, which are resistant to 5-fluorouracil, with no associated toxicity.

Herein demonstrated is an anticancer activity of cephalosporins' antibiotics, particularly ceftazidime, against DLD-1 cells and Caco-2 cells, which are colon cancer cell lines that are resistant and sensitive to 5-fluorouracil, respectively. 24 h post-treatment ceftazidime and 5-fluorouracil inhibited the proliferation of Caco-2 cells by 30% and 22%, respectively. On the other hand, the inhibitory effect of ceftazidime and 5-fluorouracil on DLD-1 cells 48 h post-treatment were similar (18% vs. control untreated cells) (Figure 6A). Interestingly, ceftazidime tested up to 150 µg/mL for up to 24 h boosted significantly (p<0.00001) and in a dose-dependent manner the metabolic activity of macrophages (RAW 264.7) (Figure 6B). This effect was restored to control untreated cells 48 h post-treatment. As expected 5-fluorouracil significantly decreased the metabolic activity of RAW 264.7 cells by 80% by 24 h of treatment, an effect that was sustained up to 48 h (Figure 6B). This data shows that, unlike 5-fluorouracil, ceftazidime's effect is devoid of toxicity and is specific to colon cancer colon cells. Further mechanistic studies showed that ceftazidime increases the percentage of DLD-1 cells in the pre-Gi phase of the cells cycle (Table 1). This effect is due to apoptosis induction as evidenced by an increase in caspase-3/7 activity 48 and 72 h post-treatment (Figure 3). On the other hand, 5-Fluorouracil induces an S-phase arrest but not apoptosis. This data confirms a more potent effect of ceftazidime through apoptosis induction against a resistant colon cancer cell line.

As shown above, a cephalosporin having a nitrogen and a first carboxyl group spaced apart by three carbon atoms, such as ceftazidime, has an anti-colon cancer effect. Accordingly, the invention relates to a new application for a cephalosporin having a nitrogen and a first carboxyl group spaced apart by three carbon atoms, e.g. selected from ceftazidime, cefpimizole, cefiderocol, and ceftolozane, preferably ceftazidime, as an agent for inducing cell death or for suppressing cell proliferation that can effectively induce colon cancer cell death or suppress colon cancer cell proliferation. Colon cancer can be treated via administration of an effective amount of ceftazidime to a patient who is need of treatment.

The features of the present invention disclosed in the specification, the claims, and/or in the accompanying figures may, both separately and in any combination thereof, be material for realizing the invention in various forms thereof.

## Claims

1. A cephalosporin for use in a method of preventing and/or treating colon cancer, wherein the cephalosporin comprises a nitrogen, preferably positively charged nitrogen, and a first carboxyl group, preferably a negatively charged carboxyl group; wherein the nitrogen and the carboxyl group of said cephalosporin are spaced apart by three carbon atoms; wherein, optionally, said cephalosporin further comprises a second carboxyl group; wherein, preferably, the cephalosporin is a third-generation cephalosporin or fourth-generation cephalosporin.

2. The cephalosporin for use according to claim 1, wherein the cephalosporin is selected from ceftazidime, cefpimizole, cefiderocol, ceftolozane, and derivatives thereof, in particular pharmaceutically acceptable salts, solvates, and hydrates thereof.

3. The cephalosporin for use according to claim 1 or 2, wherein the cephalosporin is selected from 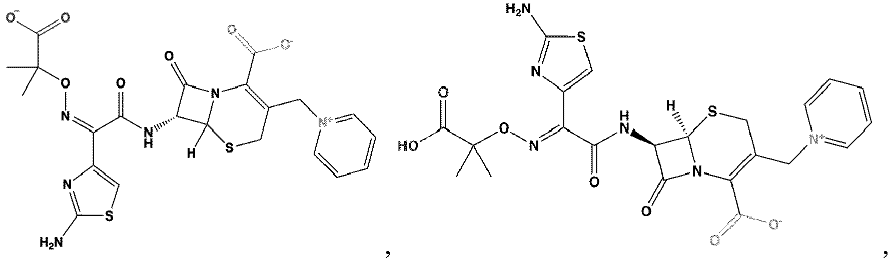 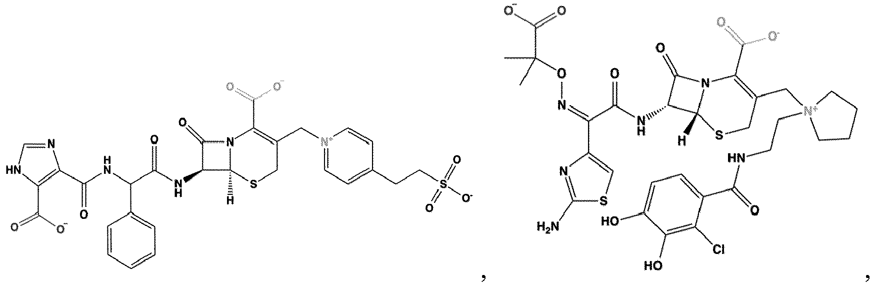 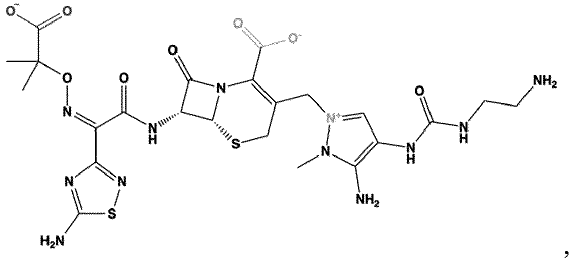 and derivatives thereof, in particular pharmaceutically acceptable salts, solvates, and hydrates thereof.

4. The cephalosporin for use according to any one of the foregoing claims, wherein said cephalosporin is selected from ceftazidime and derivatives thereof, in particular pharmaceutically acceptable salts, solvates, and hydrates thereof; preferably is ceftazidime pentahydrate.

5. The cephalosporin for use according to any one of the foregoing claims, wherein said cephalosporin is selected from 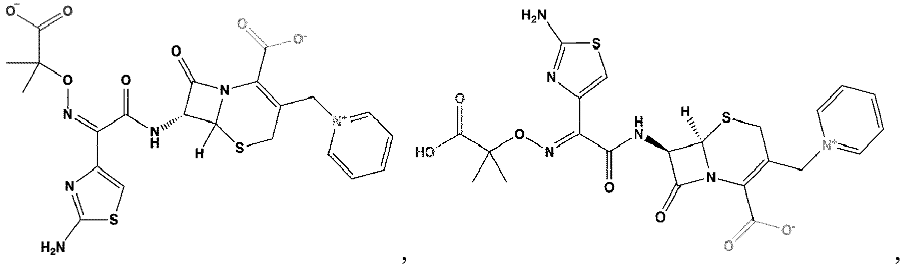 and derivatives thereof, in particular pharmaceutically acceptable salts, solvates, and hydrates thereof.

6. The cephalosporin for use according to any one of the foregoing claims, wherein said colon cancer is **characterized by** a resistance to 5-fluorouracil or sensitivity to 5-fluorouracil.

7. The cephalosporin for use according to any one of the foregoing claims, wherein said colon cancer comprises 5-fluorouracil resistant cancer cells.

8. The cephalosporin for use according to any one of the foregoing claims, wherein said method involves suppressing cell proliferation and/or inducing apoptosis in cells of said cancer, preferably in 5-fluorouracil resistant cancer cells and/or 5-fluorouracil sensitive cancer cells.

9. The cephalosporin for use according to any one of the foregoing claims, wherein said method involves suppressing cell proliferation in cells of said cancer by decreasing the metabolic activity of said cells.

10. The cephalosporin for use according to any one of the foregoing claims, wherein said method involves inducing apoptosis in cells of said cancer by inducing a pre-Gi phase and/or by increasing caspase activity, preferably caspase 3 and/or caspase 7 activity.

11. The cephalosporin for use according to any one of the foregoing claims, wherein, in said method, said cephalosporin is administered to a patient in need thereof, preferably a 5-fluorouracil naive patient or a 5-fluorouracil non-responding patient, e.g. a patient suffering from a cancer **characterized by** 5-fluorouracil resistant cells.

12. The cephalosporin for use according to any one of the foregoing claims, wherein, in said method, said cephalosporin is co-administered with a further drug, preferably further anti-colon cancer drug.

13. The cephalosporin for use according to claim 12, wherein said further drug is selected from topoisomerase inhibitors, e.g. irinotecan hydrochloride; platinum-based antineoplastic agents, e.g. oxaliplatin; compositions comprising trifluridine and tipiracil hydrochloride; mTOR inhibitors, e.g. everolimus; somatostatin analogues, e.g. lanreotide acetate; anti-CTLA4 antibodies, e.g. ipilimumab; epidermal growth factor receptor inhibitors, e.g. cetuximab and panitumumab; PD-1 receptor blockers, e.g. nivolumab and pembrolizumab; vascular endothelial growth factor inhibitors, e.g. ramucirumab, bevacizumab, and ziv-aflibercept; and oral multikinase inhibitors, e.g. regorafenib and sorafenib.

14. The cephalosporin for use according to any one of the foregoing claims, wherein, in said method, said cephalosporin is not co-administered with 5-fluorouracil.

15. A pharmaceutical composition for use in a method of preventing and/or treating colon cancer, wherein said composition comprises a cephalosporin, as defined in any one of claims 1-14, and a pharmaceutically acceptable excipient, optionally further comprises a further drug selected from topoisomerase inhibitors, e.g. irinotecan hydrochloride; platinum-based antineoplastic agents, e.g. oxaliplatin; compositions comprising trifluridine and tipiracil hydrochloride; mTOR inhibitors, e.g. everolimus; somatostatin analogues, e.g. lanreotide acetate; anti-CTLA4 antibodies, e.g. ipilimumab; epidermal growth factor receptor inhibitors, e.g. cetuximab and panitumumab; PD-1 receptor blockers, e.g. nivolumab and pembrolizumab; vascular endothelial growth factor inhibitors, e.g. ramucirumab, bevacizumab, and ziv-aflibercept; and oral multikinase inhibitors, e.g. regorafenib and sorafenib.
